Europäisches Patentamt

European Patent Office

Office européen des brevets

(11) Publication number: 0 215 987
A1

# EUROPEAN PATENT APPLICATION

(21) Application number: 85306698.3

(22) Date of filing: 20.09.85

(51) Int. Cl.4: **C12N 15/00** , C12N 9/12 , C12N 1/20 , C12N 1/18 , //(C12N1/20,C12R1:19)

(43) Date of publication of application:
01.04.87 Bulletin 87/14

(84) Designated Contracting States:
BE CH DE FR GB LI NL SE

(71) Applicant: **Director of National Institute of Agrobiological Resources**
**2-1-2, Kannondai Yatabe-machi**
**Tsukuba-gun Ibaraki-ken(JP)**

(72) Inventor: **Ikeda, Joh-e**
**907-201, 4-chome, Namiki Sakuramura**
**Nihari-gun Ibaraki-ken(JP)**
Inventor: **Hirochika, Hirohiko**
**No 416-301, 4-chome, Matsushiro**
**Yatabe-machi**
**Tsukuba-gun Ibaraki-ken(JP)**
Inventor: **Takatsuji, Hiroshi**
**No. 521-303, 5-chome, Matsushiro**
**Yatabe-machi**
**Tsukuba-gun Ibaraki-ken(JP)**

(74) Representative: **SERJEANTS**
**25, The Crescent King Street**
**Leicester, LE1 6RX(GB)**

(54) **Reverse transcriptase, gene, recombinant DNA, cell line and method.**

(57) A reverse transcriptase encoded by cauliflower mosaic virus, a cloned gene encoding for a polypeptide having the reverse transcriptase, a recombinant DNA containing the cloned gene, and living cells involving the recombinant DNA are produced from the living cells. Yeast is used as an eucaryotic host for the production of the reverse transcriptase.

EP 0 215 987 A1

## Reverse Transcriptase, Gene, Recombinant DNA, Cell Line and Method.

### DESCRIPTION

### Technical Field

The invention relates to a reverse transcriptase coded by cauliflower mosaic virus, a cloned gene encoding for a polypeptide having a reverse transcriptase activity, a recombinant DNA containing said cloned gene, and living cells involving said recombinant DNA. The invention includes a method for the production of a reverse transcriptase using living cells involving the recombinant DNA.

### Background Art

Reverse transcriptase, an enzyme which is capable of synthesizing DNA by means of RNA as a template is termed RNA dependent DNA polymerase.

In 1970, reverse transcriptase was discovered in Rausher mouse leukemia virus (abbreviated as R-MLV) by Baltimore (See: Nature, 226, 1209 (1970)), and in Raus sarcoma virus (abbreviated as RSV) by Temin and Mizutani (See: Nature, 226, 1211 (1970)).

After further investigation, it has been demonstrated that this reverse transcriptase may be encoded characteristically by RNA tumor viruses which have a tumorigenic ability and which may be classified into the family of retrovirus belonging to animal virus containing RNA as a gene, and that this reverse transcriptase is a minor component of virus particles (See: RNA tumor viruses. ed. R-Weiss et al. Cold Spring Harbor Laboratory (1982)).

The retrovirus which was first discovered in mice and chickens was later demonstrated in cats - [See: Spiegelman et al., Nature 227, 563 (1970); Hatanaka et al., Proc. Natl. Acad. Sci. U.S.A. 67, 143 (1970); Scolnik et al., Proc. Natl. Acad. Sci. U.S.A. 67, 1034 (1970)], in monkeys [See: Schlom and Spiegelman, Proc. Natl. Acad. Sci. U.S.A. 68, 1613 (1971); Scolnick et al., Science. 177, 1119 - (1972); Abrell and Gallo, J. Virol. 12, 431 (1973)] and in humans [See: Poiesz et al., Proc. Natl. Acad. Sci. U.S.A. 77, 7415 (1980); Yoshida et al., Proc. Natl. Acad. Sci. U.S.A. 79, 2031 (1982)]. From a specific mode of multiplication of the retrovirus, the retrovirus may be considered as coding for a polypeptide having a reverse transcriptase activity.

The discovery of the reverse transcriptase has resulted in an extensive advancement in gene engineering. Particularly, if it is possible to purify a messenger RNA (abbreviated as mRNA) as a gene product in higher animals and plants where identification is difficult, it is feasible to prepare by means of the reverse transcriptase a DNA complementary to said mRNA (abbreviated as c-DNA). If such c-DNA as mentioned is prepared, it can be inserted into various plasmids or into expression vectors, thereby to construct a recombinant DNA which is then transduced into and is proliferated in host cells to produce a large quantity of desired genes or gene products. Such an attempt as mentioned has already been made in the production of physiologically active proteins derived from humans such as interferones [See: Gray et al., Nature 295, 503 (1981); Nagata et al., Nature 284, 316 - (1980); Taniguchi et al., Gene 10, 11 (1980)].

Until now, purification of reverse transcriptase has been tried only from purified virus particles derived from chickens and mice, as such lines of virus to be applicable are restricted to those originating from chickens and mice that are able to supply viruses in such an amount enough to be purified to prepare reverse transcriptase as a minor component of virus particles. The reverse transcriptase now commercially available is prepared from Avian myelobrastosis virus (abbreviated as AMV) particles which may be recovered from plasma of chickens infected with viruses. Beared et al. have prepared a large quantity of reverse transcriptase - (See: J. Virol. 29, 517 (1979)) using as the starting material several tens of grams of viruses. The preparation of reverse transcriptase of a high purity and of a high quality, as well as the solution various problems such as equipment, safety, yield and materiality of virus particles, which are encountered in connection with the preparation in a large quantity of animal RNA tumor viruses as the source material for reverse transcriptase, may preferably be performed either by using as the starting material some other kinds of retroviruses of no tumorigenic activity, or by means of recombination DNA method to produce the reverse transcriptase encoded by retrovirus.

However, until now none has succeeded in preparing of a reverse transcriptase by such recombination DNA method, or prepared a reverse transcriptase from plant viruses, Cauliflower mosaic viruses which are quite different from retrovirus and have potentially tumorigenic activity.

Cauliflower mosaic virus (abbreviated as CaMV) is a plant virus which contains as a gene, a double-stranded DNA having a cyclic structure composed of about 8000 base pairs. Cauliflower mosaic virus infects plants of Brassica campestris and thereby produces mosaic specks on the leaves so that the viruses inhibit the growth of the plant. However, this virus exhibits no tumorigenic activity against animals and human beings.

Three discontinuances ($\Delta$1, $\Delta$2 and $\Delta$3) have been detected in CaMV genomic DNA. Until now, a whole base sequence has been determined for 3 lines of CaMV; Cabb-S strain (Franck et al., Cell 21, 285 (1980)), CM 1841 strain (Gardner et al., Nucleic Acid Res. 9, 2871 (1981)) and Cabb-D/H strain (Balazs et al., Gene. 19, 239 (1982)), and 7 open reading frames (abbreviated as ORF I-VII) have also been established for these lines. Based on the structure of virus genomic replication intermediates in a CaMV-infected plant cells and on the detection of reverse transcriptase-like activity linked to the virus genomic replication intermediates, the possibility has been discussed whether or not CaMV encodes for a polypeptide having a reverse transcriptase activity, as in animal retroviruses [See: Pfeiffer and Hohn et al., Cell 33, 781 (1983); Pfeiffer et al., Plant Mol. Biol. 3, 261 - (1984); Volovitch et al., EMBO Journal 3, 309 - (1984)].

THE INVENTION

The present inventors, on the presumption that CaMV contains gene encoding for a polypeptide having a reverse transcriptase activity, have tried to isolate and identify a reverse transcriptase activity from purified CaMV particles. As a result, the present inventors have succeeded in detection and identification of reverse transcriptase activity from virus particles prepared in mild conditions without resort to Triton-Urea treatment which had been applied for CaMV preparation. In a comparison of the CaMV base sequence with that of the retrovirus reverse transcriptase gene base sequence performed by computation on a computer, a base sequence similar to that of reverse transcriptase was detected in a region of the base sequence of ORF V. These facts reveal that CaMV is a plant retrovirus and CaMV bears a gene encoding a polypeptide having a reverse transcriptase activity. Since optimum temperature for growth of plant cells is 25°C to 28°C, it may be predicted that the optimum temperature for the active enzyme encoded by CaMV proliferating in plant cells at a temperature lower by about 10°C than the optimum temperature of animal cells is in a much lower range than that of enzymes which generally shows an optimum temperature of 37°C. Consequently, there may be an advantage in CaMV reverse transcriptase that the reverse transcriptase can be fully applied at such a low temperature as it is able to inhibit the DNA-or RNA-nuclease which may generally contaminate in the synthesis of c-DNA in vitro. A whole genomic size of RNA (about 8000 nucleotides) has been detected in CaMV-infected cells. As it may be considered to develop reverse transcription using the RNA as a template - (Hull and Covey et al., Nucleic Acid Res. 11, 1881 - (1983); Pfeiffer and Hohn et al., Cell 33, 781 - (1983), it is expected for CaMV reverse transcriptase to have an ability to synthesize c-DNA corresponding to 8000 nucleotides. Such characteristics observed in the CaMV reverse transcriptase are extremely useful for the synthesis of a genomic c-DNA based on a longer messenger RNA precursor derived from a higher eucaryote as the template.

As it is very difficult to prepare free CaMV particles in large quantities while keeping the reverse transcriptase active, there has been a growing demand for cloned genes encoding the reverse transcriptase and a recombinant DNA bearing such cloned genes. There has also been a requirement for living cell lines containing the recombinant DNA and for a method to produce a reverse transcriptase using such living cell lines.

A cloned gene encoding for a polypeptide having a reverse transcriptase activity has been constructed by supplying a DNA composed of a gene encoding for a polypeptide having a reverse transcriptase activity and vector DNA to be replicated in procaryotic or eucaryotic cells, the coding sequence of the gene being located down stream of a promoter sequence. The procaryotic or eucaryotic cells are transformed with a coding gene and a vector DNA so that it may produce reverse transcriptase.

Reverse transcriptase according to the invention may be prepared by aerobic cultivation in a medium suspended with procaryotic or eucaryotic cell lines, which have been transformed so that they may produce the reverse transcriptase, by inserting genes as coded to produce a polypeptide having a reverse transcriptase activity and vector DNA to be replicated in a such manner that a coding sequence of said gene be located down stream of a promoter sequence.

A cloned gene encoding for the reverse transcriptase may also be prepared from virus DNA by treating with a restriction enzyme endonuclease, the DNA region of CaMV DNA corresponding to the reverse transcriptase gene encoding a polypeptide having a reverse transcriptase activity.

As a source material of the gene encoding for the reverse transcriptase according to this invention, there is used CaMV which has been prepared by cloning onto EK-type plasmid vector (relaxed type), pBR322 a genomic DNA derived from CM 1841 strain and of which the total base sequence has been determined.

The reverse transcriptase activity was assayed by a modification of the method described in "Marcus et al., J. Virol., 14, 853 (1974)" and in "Dion et al., J. Virol., 14, 40 (1974)".

A reaction solution (0.05 ml) containing 50 mM Tris-HCl buffer (pH 7.5), 50 mM KCl, 2 mM MnCl₂, 80 μg/ml poly(rA), 10 μg/ml oligo (dG), 4 μM [³H]-dTTP (3000-4000 cpm/pmol), 5 mM DTT, 0.01% NP-40 and 100 μg/ml BSA was prepared. To this reaction solution thus prepared, a reverse transcriptase fraction was added and the resultant solution was kept constant for a definite time at 20°C. After this procedure, the enzyme activity was assayed by measuring [³H]dTMP incorporated into TCA sedimentation fraction under cooling. Enzyme activity was assayed by use of 90 μg/ml poly(rC) oligo(dG) (1:1) in the presence of 4 M [³H]dGTP (3000 to 4000 cpm/pmol), or by use of nature RNA molecules which were prepared by binding a hydrogen atom to the DNA linkage (about 200 nucleotides) complementary to the 3'-terminal of tobacco mosaic virus RNA (6000 nucleotides) as the primer, so that c-DNA synthesizing activity was determined.

A yeast (Hinnen et al., Proc. Natl. Acad. Sci. U.S.A. 75, 1929 (1978)) was used as an appropriate eucaryotic host for the production of the reverse transcriptase. As a yeast vector, a yeast host vector which has been applied by Matsubara et al. for expression of surface antigen genes of a blood serum hepatitis viruses (β-type) may be used (See: Miyanohara et al., Proc. Natl. Acad. Sci. U.S.A.. 80, 1 (1983)).

Examples of appropriate procaryote hosts include Escherichia coli and Bacillus subtilis. Escherichia coli may be used as a preferred host for DNA amplification in host cells. In a numerous EK-type plasmid vectors (either stringent or relaxed), pBR 322 and M13mp8 which are most accessible relaxed EK-type plasmids have been selected as a vector for Escherichia coli cells according to this invention.

Transformation of host cells by use of a recombinant DNA was performed in a conventional manner as will be described below. When Escherichia coli was used as the host cells, the competent cells (capable of incorporating the DNA) of the Escherichia coli were recovered at a logarithmic growth phase and were subjected to the transformation in the CaCl₂ method as known per se. When a yeast was used as host cells, these cells were subjected to the transformation in a modified Beggs method (Nature 275, 104 (1978)) (See: Method in Yeast Genetics, ed. Sherman et al., Cold Spring Harbor Laboratory (1982)).

A recombinant plasmid pAM.ORF V DNA having a reverse transcriptase activity of CaMV was obtained from yeast cells used as host cells. From this recombinant plasmid was also obtained a fragment; Xho I-Pvu II DNA encoding for a polypeptide having a reverse transcriptase activity. The total nucleotide sequence of the DNA fragment was analyzed according to the method of Maxam and Gilbert (Meth. Enzyme. 65, 499 (1980)) as well as according to 2–deoxynucleotide linkage terminal method (Smith, A.J.M. Meth. Enzym. 65, 560 - (1980) and was found identical to the nucleotide sequence of the ORF V.

DRAWINGS

Figure 1 is a flow chart showing the construction of the recombinant DNA,

Figure 2 is a phosphocellulose column chromatography of the reverse transcriptase in the crude extract,

Figure 3 is a denatured DNA cellulose column chromatography of the reverse transcriptase in the phosphocellulose fraction,

Figure 4 is a glycerol gradient centrifugation of the reverse transcriptase fraction.

EXAMPLE 1

Expression of the gene ORF V encoding for the reverse transcriptase of Cauliflower mosaic virus in yeast cells.

A recombinant DNA used for producing ORF V in living cells may be prepared in such a way, for example, either by inserting a DNA encoding ORF V down stream of a promoter sequence or by inserting a promoter sequence ahead of a cloned DNA sequence encoding ORF V. Various strains of CaMV have already been isolated, and a DNA sequence encoding ORF V may be prepared from any one of the strains mentioned. The following is an example of the procedure for expressing the ORF V in yeast cells by use of a DNA sequence from CM 1841 encoding for ORF V.

(i) Cloning of a total genomic DNA of CM 1841 strain into Bacillus subtilis cells

CM 1841 DNA was cleaved with Sal I and the cleaved product was admixed with a plasmid vector for Bacillus subtilis (pBR 322) cleaved also with Sal I, and thereby these two cleaved products were linked to each other by use of T4 DNA ligase. This linked product was then transduced into Bacillus subtilis cells. The resultant recombinant was named pBR•CM 1841.

(ii) Cloning of a DNA sequence encoding ORF V

Since the DNA sequence encoding for ORF V is apt to be cut off in the process for the preparation of pBR•CM 1841 as described above in which CM 1841 DNA cleaved with Sal I is inserted into the cleavage site of pBR 322 cleaved with Sal I, the following procedure was applied to prepare a recombinant (pBR•ORF V•Ep) containing a DNA sequence encoding for a perfect ORF V. Firstly, pBR•CM 1841 was cleaved by use of Sal I and Pvu II, and thereby a DNA fragment corresponding to the C-terminal of ORF V was taken out. The DNA fragment obtained was inserted into pBR 322 at the restriction site of Pvu II and Sal I to obtain pBR•ORF V-3'.

On the other hand, pBR•CMl841 was cleaved by use of Sal I and Cla I, thereby a DNA fragment corresponding to the middle sites of the ORF V was taken out. The DNA fragment obtained was inserted into pBR•ORF V-3' mentioned above at the restriction site of Cla I and Sal I to obtain pBR•ORF V•CP. Next, pBR•CM1841 was cleaved by use of EcoRI and Bgl II, therby a DNA fragment corresponding to the N-terminal of the ORF V was taken out. The DNA fragment obtained was inserted into the pBR•ORF V•CP mentioned above at the restriction site of EcoRI and Bgl II to obtain pBR•ORF V•EP.

(iii) Excision of a base sequence ahead the initiation codon (ATG) of ORF V, and insertion of a Sal I-recognition sequence for inserting an expression promoter

pBR•CM1841 was cleaved with Pst I, and thereby a DNA fragment containing ORF V initiation codon was taken out. The DNA fragment obtained was treated to a certain extent with Bal 31 and then was cut off with Bgl II, thereby a DNA fragment containing the initiation codon was taken out. On the other hand, pBR•ORF V•EP was cleaved by use of EcoRI and Bgl II, and thereby a DNA fragment corresponding to the N-terminal of ORF V was taken out. A mixture of the DNA fragments mentioned was inserted into a phage vector M 13 mp 8 for Bacillus subtilis at the restriction site of Sma I and EcoRI. From the recombinant viruses thus obtained, a single-stranded DNA was prepared. The single-stranded DNA was analyzed for the base sequence by the dideoxy chain termination method, and was identified as a recombinanat in which cauliflower mosaic virus DNA was cut off up to immediately before the initiation codon for ORF V. Next, double-stranded DNA propared from said recombinant viruses was cleaved with Bgl II, thereby a DNA fragment bearing the initiation codon. The DNA fragment obtained was the inserted into pBR•ORF V-CP at the restriction site of Bgl II to obtain pBR•ORF V-SB•CP.

(iv) Preparation of a recombinant pAM•ORF V expressing ORF V in yeast cells

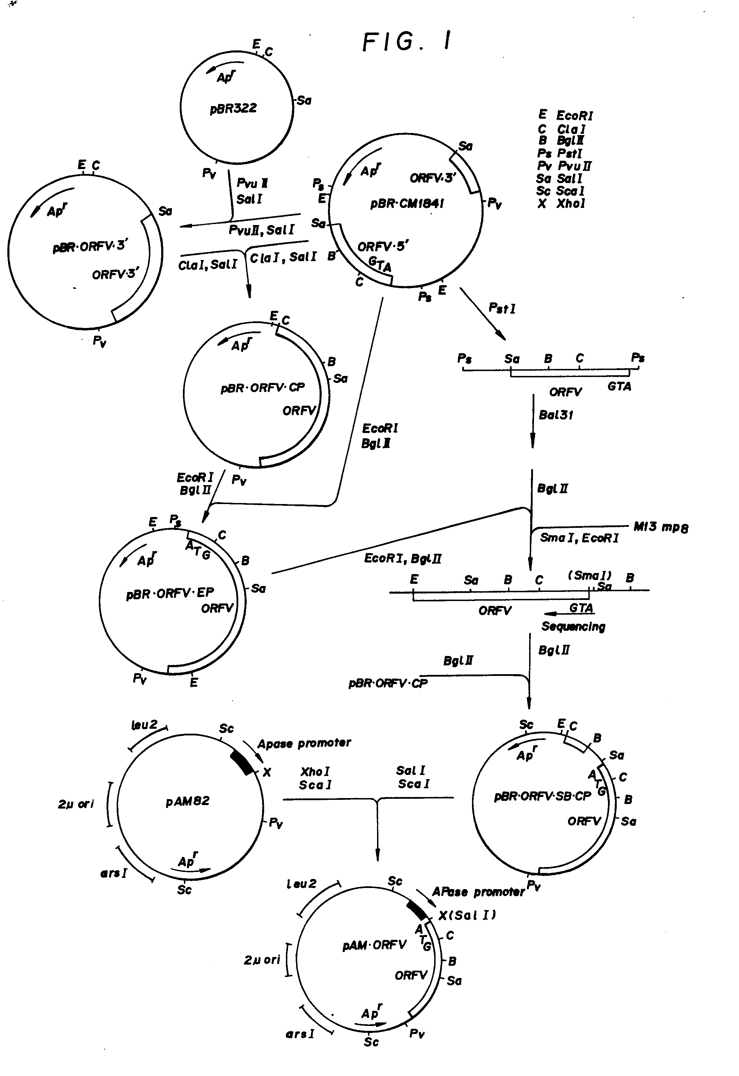

A recombinant, pAM•ORF V capable of expressing ORF V in yeast cells was prepared using a yeast expression vector, pAM 82 in the following procedure. pAM 82, a yeast-Escherichia coli shuttle vector has been reported by Miyashita in "Proc. Natl. Acad. Sci. USA. 80, 1-5 (1983)". This vector bears a marker as ars I (Stinchcomb D. T. et al., Nature 282, 39-43 (1979) and includes 2 μm Ori - (Broach, J., et al., Gene 8, 121-133 (1979), leu 2 - (Ratzkin, B. et al., Proc. Natl. Acad. Sci. U.S.A. 74, 474-491 (1979) and a promoter for yeast acid phosphatase (abbreviated as APase). APase promoter is induced just when phosphoric acid in the culture solution is shifted from of a higher concentration to of a lower concentration. pBR•ORFV•SB•CP was partially cleaved with Sal I and then was cleaved with Sca I, thereby a DNA fraction in which the cleavage occurs at a Sal I-recognition site immediately ahead the initiation codon for ORF V and which contains ORF V, was taken out. On the other hand, pAM82 was partially cleaved with Sca I and then was cleaved with Xho I, thereby a DNA fragment in which the cleavage occurs at a Sca I recognition site in a Ampicillin-resistant gene (Ap') and which contains ars I, was taken out. This DNA fragment was ligated using a ligase with the DNA fragment containing ORF V mentioned above and the ligated product was introduced into cells of yeast AH 22 strain. The resultant transformant exhibited a cell number transformed as 2/μg DNA. The recombinant plasmid obtained was named pAM•ORF V, and the yeast cell containing said recombinant plasmid - (pAM•ORF V) was named AH 22/pAMORF V. This yeast strain has been deposited in Fermentation Research Institute, Agency of Industrial Science and Technology under the deposition No. FERM P-7963 (FERM BP-851). The yeast strain was demonstrated to produce a reverse transcriptase encoded by ORF V when phoshoric acid concentration in the culture solution is lowered. Fig. 1 is a flow chart showing a construction of the recombinant DNA.

EXAMPLE 2

Expression of a gene encoding for a reverse transcriptase in yeast cells

Reverse transcriptase gene cloned onto pAM•ORF V was demonstrated in the cells of Saccharomyces cerevisiae AH22 (a leu 2 his 4 can I cir+) as host cells in the following manner. Yeast cells transformed with pAM•ORF V were cultured in a minimal nutrient liquid medium containing phosphoric acid at a higher concentration.

Cultured cells at a logarithmic phase when a concentration of 1 to $2 \times 10^5$ cells/ml was amounted, was centrifuged to precipitate the cells. The cells thus collected was suspended again and cultured in a minimal nutrient liquid medium containing phosphoric acid at a lower concentration so that the expression of the reverse transcriptase gene inserted behind the promoter for acid phosphatase may be induced. The cultivation was all conducted at 30°C and when the number of cells amounted to 2 to $3 \times 10^7$/ml, the culture solution was subjected to centrifugation so that the cells were collected. A complete synthetic medium was generally used as the minimal nutrient liquid medium containing phosphoric acid at a lower concentration, but is also applicable a yeast nitrogen base which is prepared by removing phosphoric acid by use of $MgSO_4$ and aqueous $NH_3$, followed by addition of essential amino acids and glucose. Spheloplast cells prepared in a conventional manner using Zymolase 100T were suspended in a hypotonic buffer of 50 mM Tris-HCl (pH 7.5)-1 mM DTT-1 mM PMSF containing 0.1% Triton-X100 so that the cells were destroyed. Immediately after this destruction procedure of the cells, the suspension was centrifuged for 10 minutes at 10000 x g. The resultant supernatant liquid was utilized as the reverse transcriptase fraction. The yeast cells which could not induce the expression of the reverse transcriptase gene in a minimal nutrient medium containing phosphoric acid at a lower concentration and the yeast cells which were transformed with plasmid; pAM82 devoid of the reverse transcriptase gene were processed the same manner as mentioned above to prepare supernatant liquids which were used as the control fractions. The supernatant liquids thus obtained were respectively assayed for the reverse transcriptase activity by use of two template; poly(rA)•oligo(dT) and poly(rC)•oligo-(dG). A DNA polymerase and a terminal transferase present in a crude fraction of the reverse transcriptase are also able to synthesize DNA using poly-(rA)•oligo(dT) as a template. While poly(rC)•oligo-(dG) is hardly utilized as a template by DNA polymerase. The terminal transferase does not have a substrate specificity to template DNA. Therefore, the contaminated enzymes; DNA polymerase and terminal transferase could be detected in the assay according to the invention by means of using poly-(rA)•oligo(dT) as the template, substituting dGTP for the substrate dTTP indispensable for the synthesis of the desired DNA.

Yeast AH22 strain containing plasmid pAM82 and containing a recombinant plasmid pAM•ORF V were respectively inoculated in a higher Pi medium (100 ml) which was prepared by adding histidine - (20 mg/1) and $KH_2PO_4$ (1.5 g/l) to Burkholder minimal nutriant medium (See: Basti an et al., Proc. Natl. Acad. Sci. U.S.A. 77, 4504 (1980)), and these strains were cultured at 30°C with aeration.

10 ml of the culture solution was sampled at the time when the cell concentration amounted to about $2 \times 10^5$/ml, and the sampled culture solution - (10 ml) was centrifuged to collect the cells. The cells were then suspended in a lower Pi medium - (10 ml) which was prepared by adding histidine (20 mg/ml) and KCl (1.5 g/l) to Burkholder's minimal nutrient medium, and continued to culture. The culture of yeast cells which did not induce the activation of acid phosphatase promoter were centrifuged and the collected cells were again cultured in the higher Pi· medium. When the cell number amounted to $2 \times 10^7$/ml, whole the culture solution - (10 ml) was centrifuged and the collected cells were resuspended in a spheroplast preparation solution (Zymolase 100T (100 g/ml), 1.2 M sorbitol, 50 mM phosphate buffer (pH 7.2), 1 mM dithiothreitol) (3 ml) and allowed to stand for 30 minutes at 30°C. The resultant solution containing spheroplasts thus obtained was centrifuged at 2500 rpm for 5 minutes. The collected spheroplasts were suspended in an ice-cooled hypotonic solution for degradation thereof (0.1% Triton X-100, 1 mM dithiothreitol (DTT), 1 mM phenylmethylsulfonylfluoride (PMSF), 50 mM Tris-HCl buffer (pH 7.5) (1 ml) and was kept for 10 minutes at 0°C, followed by centrifugation at 7000 rpm for 10 minutes. The resultant supernatant solution was assayed for reverse transcriptase activity. Protein concentration was assayed by a modified Bradford method - (Bradford, Anal. Biochem., 72, 249 (1976) using Protein Assay kit (Bio-Rad).

Yeast cells containing pAM•ORF V (plasmid inserted with ORFV) and yeast cells containing pAM82 (plasmid not inserted with ORFV) were cultured in such a manner as mentioned above in either inducing (positive) or not inducing (negative) condition for the production of reverse transcriptase and processed to prepare a crude solution containing the reverse transcriptase. The reverse transcriptase activity (RNA dependent DNA synthesizing activity) in the preparated solution was assayed using as the template poly(rA)•oligo(dT) and poly-(rC)•oligo(dG).

In the case of poly(rA)•oligo(dT) as the template, incorporation of [³H]dTMP into the acid-insoluble fractions was measured. In the case of poly(rC)•oligo(dG) as the template, incorporation of [³H]dGMP into the acid-insoluble fractions was measured. The reverse transcriptase activity was expressed in terms of the incorporation of [³H]-dTMP or [³H]dGMP as mentioned. Terminal transferase which is often existing together with the reverse transcriptase in the isolated solution was assayed in terms of the quantity of [³H]dGMP incorporated into acid insoluble fraction by use of poly(rA)•oligo(dT) as the template.

Crude isolated solutions corresponding to protein as 9 µg were subjected to the assay for the enzyme activity, which was conducted in the reaction solution as mentioned above from 30 minutes at 30°C. The enzyme activity was expressed in terms of the specific activity (See: Table 1), which corresponds to the DNA synthesizing ability (as pmol of deoxymononucleotide) per 1 mg of protein. Increase in the activity of RNA dependent DNA synthesizing enzyme was observed only in the yeast cells containing plasmid pAM•ORF V, in which the enzyme production was induced therein. In view of template specificity, the increase in the enzyme activity may attributed to the production of the reverse transcriptase which has been coded by ORF V. Such an induction of the reverse transcriptase as mentioned above clearly demonstrated that this enzyme corresponds to the polypeptide coded by ORFV gene of cauliflower mosaic virus which has been inserted down stream of acid phosphatase promoter derived from the plasmid PAM82. The induced enzyme was fully resistant to N-ethyl-maleimide (1 mM) which is known as a DNA polymerase inhibitor. There was not observed any increase by induction in terminal transferase activity.

## Table 1

Expression of gene encoding for reverse transcriptase in yeast cells

| Plasmid | Induc-tion | Specific activity | | |
|---------|------------|------------------------------------|------------------------------------|------------------------------------|
| | | poly(rA)-oligo(dT) [³H]dTTP | poly(rC)-oligo(dG) [³H]dGTP | poly(rA)-oligo(dT) [³H]dGTP |
| pAM82 | – | 60.2 | 14.3 | – |
| pAM82 | + | 12.2 | 11.1 | 10.8 |
| pAM:ORFV | – | 34.4 | 14.2 | – |
| pAM:ORFV | + | 486 | 191 | 13.0 |

## EXAMPLE 3

Purification of the reverse transcriptase produced by use of yeast cells containing recombinant plasmid pAM•ORFV

(1) The yeast cells containing pAM•ORF V - (AH22/pAM•ORFV) (deposited under FERM BP-851) was cultured under the conditions as described in the Example 2 as that the production of the reverse transcriptase may be induced. 1.5 liter of the cultured solution (total cell number: about 3×10¹⁹) obtained was used as the starting material. The culture solution was centrifuged in a conventional manner so that the cells of the yeast were collected. The collected cells were resuspended in a preparing solution for spheroplast (100 ml) and allowed to stand under warming at 30°C for 30 minutes so that spheroplast were prepared. The resulting solution containing spheroplasts were centrifuged and the spheroplasts thus collected were resuspended in a hypotonic solution for degradation of spheroplasts (50 ml) which had previously been ice-cooled. The suspension obtained was kept at 0°C for 10 minutes, and then was centrifuged (7000 rpm) at 4°C for 10 minutes so that there was prepared a supernatant solution (51 ml) containing a total amount of 90 mg of protein. The following procedure was carried out at 4°C in order to purify the enzyme preparation.

(2) Fractional purification of a crude solution containing the reverse transcriptase by means of phosphocellulose column chromatography

As phosphocellulose preparation, P 11 (a product of Watmann) was used. The phosphocellulose P 11 activated in a conventional manner was equilibrated in the buffer A containing 50 mM Tris-HCl - (pH 7.5), 2 mM mercaptoethanol, 0.2 mM EDTA, 0.01% NP-40, 20% (w/v) glycerol. A crude solution (50 ml containing the reverse transcriptase (protein concentration: 1.76 mg/ml) was charged at a flow rate of 20 ml/hour onto a column (2 cm in diameter, 3 cm in height) filled with phosphocellulose previously equilibrated in the buffer A containing 50 mM KCl. After washing with 20 ml of buffer A containing 50 mA, the phosphocellulose in the column was subjected to a gradient elutition (total elution volume: 100 ml) with the buffer A, eluting with a gradient of increasing linearly the KCl concentration from 50 mM to 800 mM so that the reverse transcriptase adsorbed on the phosphocellulose was eluted out. The eluate was collected each in a fraction of 0.8 ml. A sample (0.005 ml) was withdrawn from each fractions of the eluate and was assayed for the reverse transcriptase activity using as the template poly(rA)•oligo(dT) according to the procedure described above.

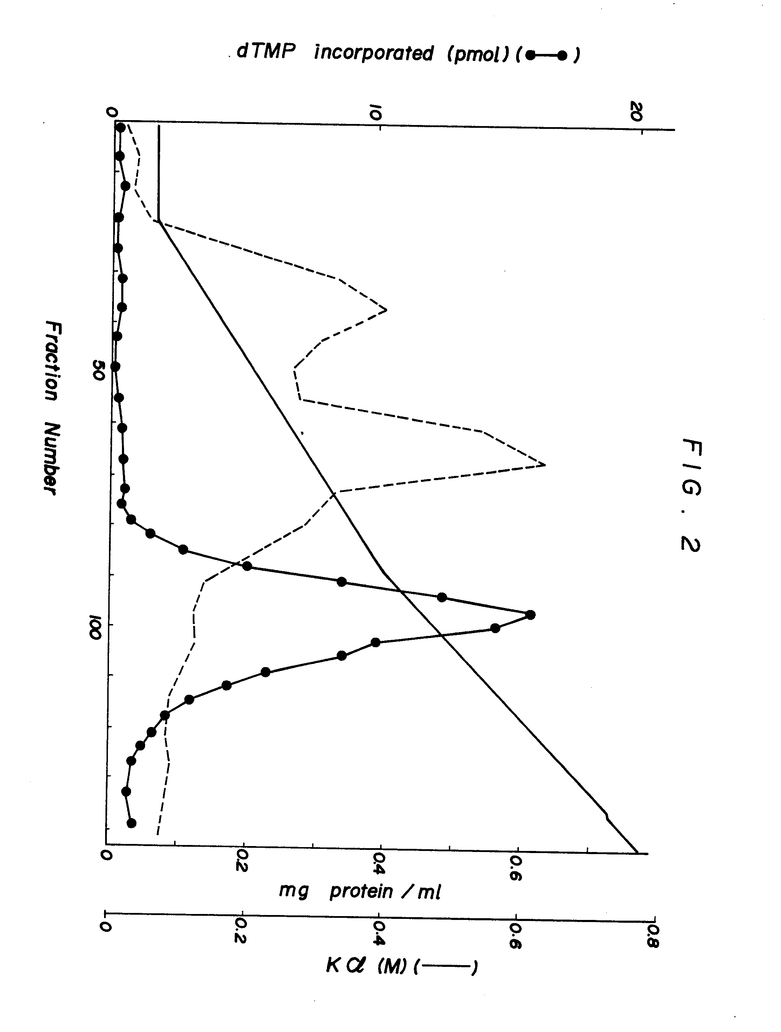

The enzyme activity determined in the reaction at 20°C for 1 hour, protein content and KCl concentration in the fractions are shown in Fig. 2. As apparent from the Fig. 2, the enzyme was eluted out in the eluate (buffer solution A) containing 0.35 M to 0.58 M KCl, exhibiling a single peak of the activity at the concentration of 0.45 M KCl. Fractions Nos. 88 to 112 (total volume: 10 ml, total amount of protein: 2.52 mg) were termed phosphocellulose fraction.

(3) Fractional purification of the phosphocellulose fraction containing the reverse transcriptase by single-stranded DNA cellulose column chromatography.

A calf thymus DNA processed with a mixture of phenol and chloroform was treated with an alkali solution so that single-stranded DNA was obtained. The single-stranded DNA was processed together with cellulose CF 11 (Watmann) in a conventional manner, to prepare a DNA cellulose capable of containing 1 mg DNA per 1 ml volume in a column. The single-stranded DNA cellulose saturated with the buffer A containing 1M KCl, was filled in a column (1.2 cm in diameter, 2.5 cm in height) which was washed 10 times the column capacity of the buffer solution A containing 1M KCl, followed by equilibration with the buffer solution A containing 20 mM KCl.

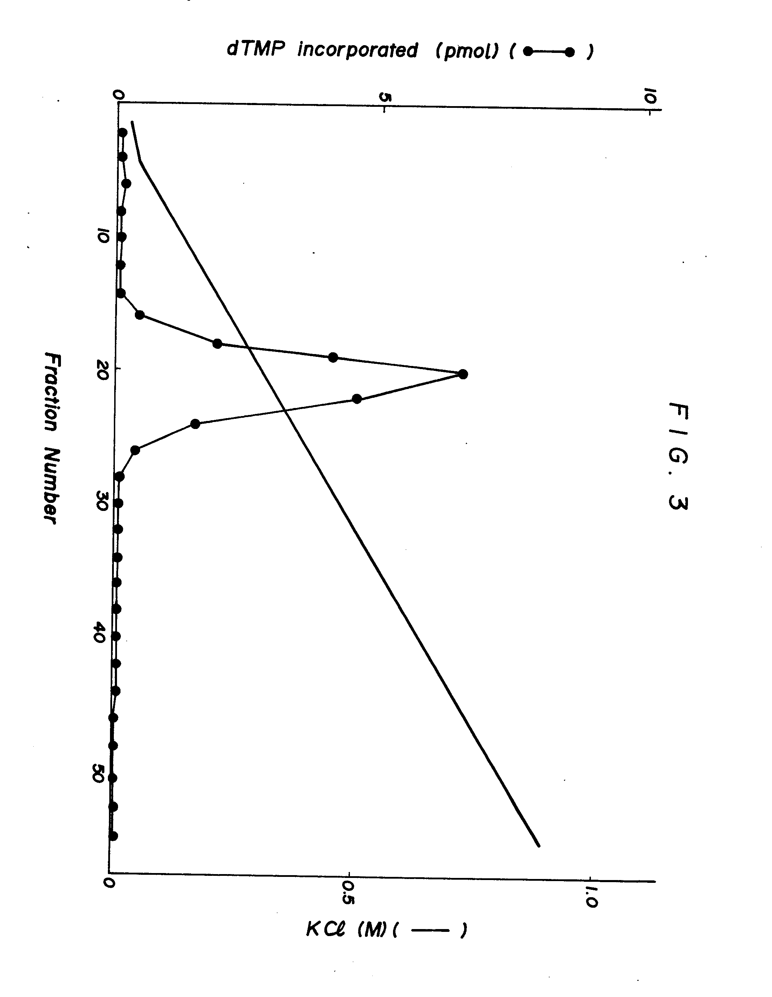

The phosphocellulose fraction (total amount of 2.4 mg of protein) (9.5 ml) was dialyzed against the buffer solution A containing 20 mM KCl. The dialyzing solution (500 ml) was replaced twice at an interval of 2 hours. The dialysis was performed in an ice-batch. When the KCl concentration in the phosphocellulose fraction became below 50 mM, the phosphocellulose fraction was charged at a flow rate of 10 ml/hour onto a column filled with a single-stranded cellulose. The single-stranded cellulose filled in the column was washed with the buffer solution A (50 ml) containing 20 mM KCl, and then was subjected to a gradient elution (total elution volume: 30 ml) with the buffer solution A, eluting with a gradient of increasing linearly its KCl concentration from 20 mM to 1M so that the reverse transcriptase adsorbed on the single-stranded cellulose was eluted. The elute was collected wach in a fraction of 0.5 ml. A sample (0.003 ml) withdrawn from each fractions of the eluate was assayed for the reverse transcriptase activity using as the templete poly(rA)•oligo(dT) according to the procedure described above. The enzyme activity determined in the reaction at 20°C for 1 hour and KCl concentration are shown in Fig. 3. As apparent from the Fig. 3, about 80% of the total enzyme activity was eluted out in the eluate (buffer solution A) containing 0.2M to 0.42M KCl, exhibiting a single peak (maximum actvity) at the concentration of 0.3M KCl. Fraction Nos. 19 to 23 (total volume: 2.5 ml, total amount of protein 0.41 mg) were termed the single-stranded DNA cellulose fraction.

(4) Purification of the single-stranded DNA cellulose fraction containing the reverse transcriptase by means of glycerol density-gradient centrifugation method

The single-stranded DNA cellulose fraction (2.4 ml) was diluted two times with the buffer solution A without glycerol, and immediately was charged onto a column (volume: 1 ml) filled with phosphocellulose previously equilibrated in the buffer A containing 10% (w/v) glycerol. The buffer solution A (glycerol concentration: 10%) containing 1M KCl was passed through the column so that the enzyme, reverse transcription was eluted. The eluate was collected each in a fraction of 0.1 ml. The fractions rich in the reverse transcriptase (total: 0.4 ml) was served as the concentrated enzyme fraction in the following procedure.

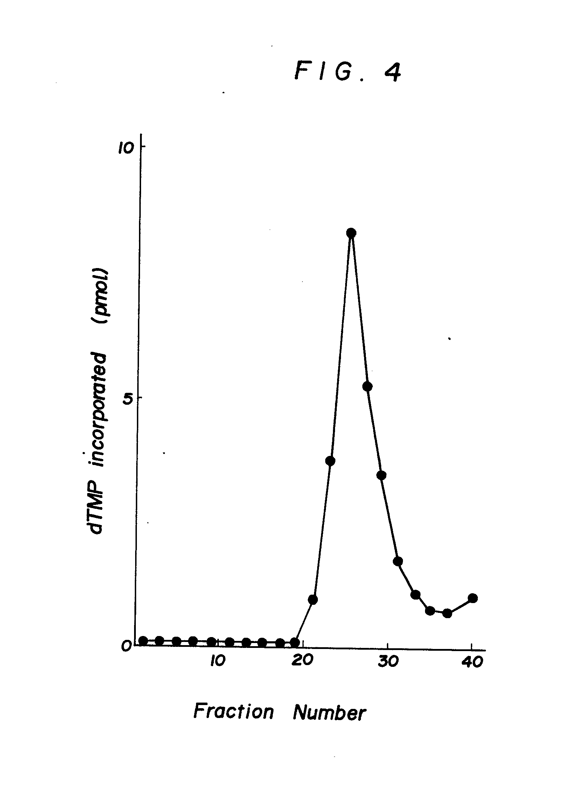

The concentrated enzyme fraction (0.1 ml) was placed on a phase of a glycerin density gradient which was prepared from the buffer solution A (5 ml) containing 1M KCl as linearly increasing the glycerine concentration from 15% to 35% and the combined phases were centrifuged using Hitachi RPS 50-2 rotor at 48000 rpm for 24 hours. When the centrifugation was finished, the centrifuged solution was collected from the bottom of the centrifuge tube each in a fraction of 0.12 ml. A sample (0.002 ml) withdrawn from each fractions was assayed for the reverse transcriptase activity using as the template poly(rA)•oligo(dT) according to the procedure as described hereinbefore (the reaction for assay of the enzyme was carried out at 20°C for 1 hour). The results are as shown in Fig. 4. There was observed a single peak for the enzyme activity in the sedimentation fractions corresponding to the molecular weight from 70000 dalton to 80000 dalton (the molecular weight was decided based on and compared to the sedimentation of cytochrome C, aldolase and catalase as the marker which were centrifuged under the same conditions). On the basis of the base number of ORFV - (2043 nucleotides), the molecular weight of the polypeptide to be coded for may be estimated as having a molecular weight of about 79000 dalton.

The enzyme fraction Nos. 23 to 28 were combined and dialyzed against the buffer solution A containing 0.1M KCl, which was termed glycerol density gradient centrifugation fraction. A summarized process for the purification of the reverse transcriptase produced by yeast cells - (AH22/pAM•ORFV) containing the recombinant plasmid pAM•ORFV is shown in Table 2. One unit of the reverse transcriptase activity (in the Table 2) is referred to as the enzyme activity which incorporates 1 n mol of dTMP into acid insoluble fraction at 20°C for 1 hour. But, for crude isolated solution the enzyme activity was assayed by the reaction for 15 minutes at 20°C and the activity assayed was converted to and expressed in terms of the one as for 1 hour of reaction.

The enzyme yield in the final product, the glycerol density gradient centrifugation fraction was 20%, and the enzyme has been purified about 4000 times in terms of specific activity.

Table 2

Purification of the reverse transcriptase coded by ORFV gene of CaMV which is expressed in yeast cells

| Fraction | Total amount of protein (mg) | Total unit number of activity | Specific activity (unit/mg protein) |
|---|---|---|---|
| Crude extract        fraction | 90 | 105.3 | 1.18 |
| Phosphocellulose fraction | 2.52 | 37.1 | 15.1 |
| Single-stranded DNA-cellulose fraction | 0.41 | 23.4 | 74.9 |
| Glycerol density gradient centrifugation fraction | $\leq$0.005 | 20.2 | $\geq$4032 |

EXAMPLE 4

The template specificity of the enzyme produced in the yeast cells (AH22/pAM•AM•ORFV) - (FERM BP-851) was examined at various steps in the purification procedure so that the polypeptide encoded by ORFV gene was confirmed to have a reverse transcriptase activity.

The reverse transcriptase is characterized in the ability to utilize as the template poly(rA)•oligo-(dT) as well as poly(rC)•oligo(dG) and poly(rCm)-•oligo(dG) (polyribomethylcytosin•oligodeoxyglycine). Crude extraction fraction, phosphocellulose fraction, single-stranded DNA cellulose fraction and glycerol density gradient centrifugation fraction which were prepared in each step of the same purification procedure as described in the Example 3, were assayed for the reverse transcriptase activity in the presence of 3 kinds of templates. The reaction solution (0.05 ml), the template (90 $\mu$g/ml), the enzyme (9 $\mu$g -5 ng) were allowed to react in the same manner as mentioned hereinbefore at 20°C for 1 hour. The enzyme activity assayed is expressed in terms of the incorporation (n mol) into the acid insoluble fraction of dNMP per 1 mg protein (See: the Table 3).

## Table 3

Template specificity of the enzyme at various steps of purification

| Fraction | Specific activity (nmol/mg protein) | | |
|---|---|---|---|
| | poly(rA)-oligo(dT) | poly(rC)-oligo(dG) | poly-(rCm)-oligo(dG) |
| Crude extract fraction | 1.21 | 0.418 | nd |
| Phosphocellulose fraction | 20.1 | 2.82 | 1.14 |
| Single-stranded DNA-cellulose fraction | 87.7 | 13.7 | 9.23 |
| Glycerol density gradient centrifugation fraction | 4050 | 1030 | 1050 |

The optimum temperature for the reverse transcriptase activity of each fractions obtained at various steps of the purification was 20°C. The enzyme activity of each fractions measured at 15°C and 30°C was 70% and 50% respectively of the activity of the same fractions measured at 20°C. The enzyme activity in the reaction at 20°C showed a linear increasement for 3 hours, and then it continued to increase with a different rate. Only the enzyme activity in the crude extract fraction increased linearly for 20 minutes after the starting of the reaction and then it was held constant.

The enzyme fraction obtained by glycerol density-gradient centrifugation has been demonstrated as purified 46 times in the assay using poly(rA)•oligo(dT), 75 times in the assay using poly(rC)•oligo(dG), and 113 times in the assay using poly(rCm)•oligo(dG), each comparing with the enzyme fraction obtained by use of single-stranded DNA cellulose column. In the enzyme fraction obtained by glycerol density-gradient centrifugation method, there was completely maintained all the template specificity characteristic for the reverse transcriptase.

As apparent from the detailed description, there could be isolated as a purified form an enzyme identified as the reverse transcriptase so far as ORFV gene is induced to be expressed in yeast cells transformed with pAM•ORFV gene.

A method for production of the reverse transcriptase encoded by cauliflower mosaic viruses was thus established by use of yeast cells.

## Claims

1. A reverse transcriptase encoded by cauliflower mosaic virus.

2. A cauliflower mosaic virus gene encoding a polypeptide having a reverse transcriptase activity.

3. A recombinant DNA which is composed of a gene derived from cauliflower mosaic viruses encoding a polypeptide having a reverse transcriptase activity and of a vector DNA to be replicated in procaryotic or eucaryotic cells, and wherein the coding sequence of the gene is located down stream of a promotor sequence.

4. A living cell line of eucaryote or procaryote each transformed by a recombinant DNA which is composed of cauliflower mosaic virus gene encoding a polypeptide having a reverse transcriptase activity, and of a vector DNA to be replicated in eucaryotic or procaryotic cells, and wherein the coding sequence of the gene is located down stream of a promoter sequence.

5. A method for production of reverse transcriptase which comprises cultivating in a medium procaryotic or eucaryotic cells each transformed with a recombinant DNA which is composed of a gene encoding a polypeptide having a reverse transcriptase activity and of a vector DNA to be replicated in procaryotic or eucaryotic cells, the coding sequence of the gene being located down stream of a promoter sequence.

6. A method for production of reverse transcriptase as claimed in claim 5, wherein the gene is prepared by messenger RNA derived from procaryotic or eucaryotic cells.

7. A method for production of reverse transcriptase as claimed in claim 5, wherein the eucaryotic cells are cells infected with animal or plant viruses each encoding for the polypeptide having a reverse transcriptase activity, or are cells injected with RNA or DNA of the viruses.

8. A method for production of reverse transcriptase as claimed in claim 6, wherein the procaryote belongs to the genus of Escherichia.

9. A method for production of reverse transcriptase as claimed in claim 8, wherein the bacterium of the genus Escherichia is Escherichia coli.

10. A method for production of reverse transcriptase as claimed in claim 6, wherein the eucaryote belongs to a yeast of the genus of Saccharomyces.

11. A method for production of reverse transcriptase as claimed in claim 10, wherein the yeast of the genus of Saccharomyces is Saccharomyces cerevisiae.

FIG. I

F I G . 2

F I G . 3

dTMP incorporated (pmol) (●——●)

KCℓ (M) ( —— )

Fraction Number

0 215 987

# FIG. 4

Fraction Number

| | **DOCUMENTS CONSIDERED TO BE RELEVANT** | | Page 2 |
|---|---|---|---|
| Category | Citation of document with indication, where appropriate, of relevant passages | Relevant to claim | CLASSIFICATION OF THE APPLICATION (Int. Cl.4) |
| A | NUCLEIC ACIDS RESEARCH, vol. 12, no. 3, 1984, pages 1517-1528; Y. MARCO et al.: "Intracellular forms of viral DNA consistent with a model of reverse transcriptional replication of the cauliflower mosaic virus genome" <br> * Page 1527 * | 1-11 | |
| A | PROC. NATL. ACAD. SCI. USA, vol. 82, August 1985, pages 4944-4948; N. TANESE et al.: "Expression of enzymatically active reverse transcriptase in Escherichia coli" <br> * Abstract * | 1-11 | |
| A | CHEMICAL ABSTRACTS, vol. 96, no. 1, 4th January 1982, page 459, no. 4948v, Columbus, Ohio, US; & JP - A - 81 87 600 (GAN KENKYUKAI FOUNDATIONS) 16-07-1981 <br> * Abstract * | 1-11 | TECHNICAL FIELDS SEARCHED (Int. Cl.4) |
| D,A | PROC. NATL. ACAD. SCI. USA, vol. 80, January 1983, pages 1-5; A. MIYANOHARA et al.: "Expression of hepatitus B surface antigen gene in yeast" <br> * Abstract * | 7,10, 11 | |
| | ---     -/- | | |

The present search report has been drawn up for all claims

| Place of search | Date of completion of the search | Examiner |
|---|---|---|
| THE HAGUE | 15-05-1986 | MADDOX A.D. |

| DOCUMENTS CONSIDERED TO BE RELEVANT | | | Page 3 |
|---|---|---|---|
| **Category** | **Citation of document with indication, where appropriate, of relevant passages** | **Relevant to claim** | **CLASSIFICATION OF THE APPLICATION (Int. Cl.4)** |
| A | PATENTS ABSTRACTS OF JAPAN, vol. 9, no. 233 (C-304) [1956], 19th September 1985; & JP - A - 60 91 981 (NIHON SEIBUTSU KAGAKU KENKYUSHO) 23-05-1985<br><br>- - - - - | | |
| | | | **TECHNICAL FIELDS SEARCHED (Int. Cl.4)** |

The present search report has been drawn up for all claims

| Place of search | Date of completion of the search | Examiner |
|---|---|---|
| THE HAGUE | 15-05-1986 | MADDOX A.D. |

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | GENE, vol. 36, no. 3, 1985, pages 271-279, Elsevier Science Publishers; V. ZIEGLER et al.: "Immunological detection of cauliflower mosaic virus gen V protein produced in engineerd bacteria confected plants" * Whole document * | 1-11 | C 12 N 15/00<br>C 12 N 9/12<br>C 12 N 1/20<br>C 12 N 1/18 //<br>(C 12 N 1/20<br>C 12 R 1:19 ) |
| D,X | CHEMICAL ABSTRACTS, vol. 100, no. 15, 9th April 1984, page 327, no. 117971s, Columbus, Ohio, US; M. VOLOVITCH et al.: "RNA-dependent DNA polymerase activity in cauliflower mosaic virus-infected plant leaves", & EMBO J. 1984, 3(2), 309-14 * Abstract * | 1,2 | |
| D,A | Idem | 1-11 | **TECHNICAL FIELDS SEARCHED (Int. Cl.4)** |
| A | NATURE, vol. 305, October 1983, pages 827-829, Macmilan Journals Ltd.; H. TOH et al.: "Sequence homology between retroviral reverse transcriptase and putative polymerases of hepatitis B virus and cauliflower mosaic virus" * Whole document * <br><br> --- -/- | 1-11 | C 12 N |

The present search report has been drawn up for all claims

| Place of search | Date of completion of the search | Examiner |
|---|---|---|
| THE HAGUE | 15-05-1986 | MADDOX A.D. |